# EUROPÄISCHE PATENTANMELDUNG

(11) **EP 1 519 303 A2**
(43) Veröffentlichungstag der Anmeldung: **30.03.2005**
(21) Anmeldenummer: 04090335.3
(22) Anmeldetag: 27.08.2004
(51) Int. Cl.: G06K 9/66, A61B 5/04, G06F 17/00, G06N 3/04

(54) **Vorrichtung zur Klassifikation physiologischer Ereignisse**

(30) Priorität: 29.09.2003 US 674280
(71) Anmelder: Biotronik GmbH & Co. KG, 12359 Berlin (DE)
(72) Erfinder: Schomburg, Richard A., Hillsboro Oregon 97123-9048 (US)
(74) Vertreter: Eisenführ, Speiser & Partner

(57) **Zusammenfassung**

Eine erfindungsgemäße Vorrichtung zur Klassifikation physiologischer Ereignisse anhand von die physiologischen Ereignisse darstellenden oder repräsentierenden physiologischen Signalen mit Hilfe eines probabilistischen neuronalen Netzwerks 5 umfasst ein zum Empfang eines das physiologische Signal repräsentierenden Wertesatzes ausgestaltetes probabilistisches neuronales Netzwerk 5, das eine Anzahl physiologische Ereignisse repräsentierender Ereignisklassen enthält, die jeweils durch eine Anzahl von Vergleichswerten bestimmt sind, und das dazu ausgestaltet ist, anhand des Vergleichs des Wertesatzes mit den Vergleichswerten eine Zuordnung des von dem Wertesatz repräsentierten physiologischen Signals zu einer der Ereignisklassen vorzunehmen, und eine mit dem probabilistischen neuronalen Netzwerk 5 verbundene Aktualisierungseinheit 10 zum Aktualisieren der Vergleichswerte einer Ereignisklasse anhand des Wertesatzes mindestens eines physiologischen Signals, das bei einer vorangegangen Zuordnung dieser Ereignisklasse zugeordnet worden ist.

## Beschreibung

Die vorliegende Erfindung betrifft eine Vorrichtung zur Klassifikation physiologischer Ereignisse, insbesondere physiologischer Ereignisse wie zum Beispiel Herzreaktionen anhand eines Elektrokardiogramms.

Physiologische Ereignisse rufen physiologische Signale hervor oder stellen selbst Signale dar, anhand derer sie klassifiziert werden können. Das Klassifizieren von physiologischen Ereignissen bzw. Signalen ist insbesondere bei implantierbaren medizinischen Geräten, wie etwa Herzschrittmachern oder implantierbaren Defibrillatoren nützlich, um behandlungsbedürftige Ereignisse von solchen, die nicht behandlungsbedürftig sind, oder Ereignisse, für die unterschiedliche Behandlungen angezeigt sind, voneinander zu unterscheiden. Anhand der Klassifikation wird das implantierbare medizinische Gerät in die Lage versetzt, selbständig die ggf. notwendige Behandlung auszulösen.

Bisherige Vorrichtungen zur Klassifikation von physiologischen Ereignissen, insbesondere von intrakardialen Ereignissen, in implantierbaren medizinischen Geräten beruhen im Wesentlichen auf einem Filtern der Signalform sowie auf dem Vorsehen eines Schwellenwertes oder mehrerer Schwellenwerte in Kombination mit einer Zeitanalyse des Über-/Unterschreitens des Schwellenwertes bzw. der Schwellenwerte.

Um mit den bisherigen Vorrichtungen eine akzeptable Empfindlichkeit auf die Signale von physiologischen Ereignissen sowie eine akzeptable Unterscheidbarkeit von Ereignissen zu erzielen, ist es notwendig, während des Herzzyklus, in den ein Ereignis fällt, das Aufnehmen von weiteren physiologischen Signalen auszusetzen. Dieses Aussetzen schließt jedoch das zuverlässige Erkennen verschiedener wichtiger Ereignisklassen von intrakardialen Ereignissen sowie deren wirksame Behandlung aus, so bspw. eine anormale Beziehung zwischen den beiden Herzkammern.

Es ist daher Aufgabe der vorliegenden Erfindung, eine verbesserte Vorrichtung zur Klassifikation von physiologischen Ereignissen, insbesondere intrakardialer Ereignisse, zur Verfügung zu. stellen, welche die genannten Nachteile zu überwinden hilft.

Diese Aufgabe wird durch eine Vorrichtung zur Klassifikation physiologischer Ereignisse nach Anspruch 1 gelöst. Die abhängigen Ansprüche enthalten vorteilhafte Ausgestaltungen der Erfindung.

Eine erfindungsgemäße Vorrichtung zur Klassifikation physiologischer Ereignisse anhand von die physiologischen Ereignisse darstellenden oder repräsentierenden physiologischen Signalen mit Hilfe eines probabilistischen neuronalen Netzwerks umfasst:
- ein zum Empfang eines das physiologische Signal repräsentierenden Wertesatzes ausgestaltetes probabilistisches neuronales Netzwerk, das eine Anzahl physiologische Ereignisse repräsentierender Ereignisklassen enthält, die jeweils durch eine Anzahl von Vergleichswerten bestimmt sind, und das dazu ausgestaltet ist, anhand des Vergleichs des Wertesatzes mit den Vergleichswerten eine Zuordnung des von dem Wertesatz repräsentierten physiologischen Signals zu einer der Ereignisklassen vorzunehmen, und
- eine mit dem probabilistischen neuronalen Netzwerk verbundene Aktualisierungseinheit zum Aktualisieren der Vergleichswerte einer Ereignisklasse anhand des Wertesatzes mindestens eines physiologischen Signals, das bei einer vorangegangen Zuordnung dieser Ereignisklasse zugeordnet worden ist.

Als physiologisches Signal soll hierbei auch ein vor dem Eingeben in die Klassifikationseinheit aufbereitetes, bspw. normiertes, gefiltertes, justiertes etc. physiologisches Signal angesehen werden. Das physiologische Signal kann selbst als das physiologische Ereignis angesehen werden oder durch das physiologische Ereignis hervorgerufen sein.

Die vorliegende Erfindung, die sich insbesondere zum Einsatz in einem implantierbaren medizinischen Gerät, bspw. einem Herzschrittmacher oder Defibrillator, eignet, beruht auf den folgenden Erkenntnissen:

Zum Klassifizieren physiologischer Ereignisse anhand sie repräsentierender physiologischer Signale eignen sich probabilistische neuronale Netzwerke. In solch einem probabilistischen neuronalen Netzwerk erfolgt das Klassifizieren anhand eines Vergleiches eines Satzes von Koeffizienten als Wertesatz, welche das physiologische Signal repräsentieren, mit einem Satz Vergleichskoeffizienten als Vergleichswerte, welche die für ein bestimmtes physiologisches Ereignis, d.h. für die Ereignisklasse des Signals, typische Signalform repräsentieren. Das Signal bzw. das Ereignis, auf dem das Signal beruht, wird dann derjenigen Ereignisklasse zugeordnet, bei der die größte Ähnlichkeit zwischen dem Satz von Koeffizienten und den Vergleichskoeffizienten vorliegt.

Ein Satz von Vergleichskoeffizienten ist im probabilistischen neuronalen Netzwerk jeweils einem Knoten zugeordnet. Einer Ereignisklasse können auch mehrere Sätze von Vergleichskoeffizienten zugeordnet sein, wobei dann jeder dieser Sätze einem eigenen Knoten zugeordnet ist, so dass für diese Ereignisklasse eine Anzahl Knoten (ein Knoten-Cluster) vorhanden ist.

Bisher wurden die Vergleichkoeffizienten für ein Ereignis derart festgelegt, dass vorab die Koeffizienten von Signalen mit der für die jeweilige Ereignisklasse typischen Signalform ermittelt und einem Knoten als Vergleichskoeffizienten zugeordnet wurden. Bei implantierbaren medizinischen Geräten kann das Festlegen der Vergleichskoeffizienten bspw. während oder kurz nach der Implantation erfolgen.

Die beschriebene Art, die Vergleichskoeffizienten festzulegen, hat jedoch zur Folge, dass die Vergleichskoeffizienten für die Zukunft festgelegt sind. Wenn sich die typische Signalform von Signalen, die physiologische Ereignisse darstellen oder auf solche zurückgehen, im Laufe der Zeit allmählich ändert, kann dies dazu führen, dass die Vergleichkoeffizienten der entsprechenden Ereignisklasse die zugehörige Signalform nicht mehr adäquat beschreiben.

Es gibt Ereignisklassen, für die absolut festgelegte Vergleichskoeffizienten nicht nötig sind, oder bei denen die Änderung der Signalform ein signifikantes Verhalten darstellt. Für diese Ereignisklassen können die vorab festgelegten Koeffizienten daher zu einer Ungenauigkeit in der Klassifikation führen, was das effektive Unterscheiden zwischen Signalen, die zu einer entsprechenden Ereignisklasse gehören und solchen, die nicht dazu gehören, erschwert oder gar unmöglich macht. Um das Verringern der Klassifikationsgenauigkeit für derartige Ereignisklassen zu vermieden, umfasst die erfindungsgemäße Klassifikationsvorrichtung daher eine Aktualisierungseinheit, mit deren Hilfe die Vergleichkoeffizienten aktualisiert und so an allmähliche Änderungen in den typischen Signalformen von physiologische Ereignisse repräsentierenden Signalen der entsprechenden Ereignisklassen angepasst werden. Auf diese Weise kann eine effektive Unterscheidung zwischen Signalen, die zu einer Ereignisklasse gehören uns solchen, die nicht dazu gehören dauerhaft, aufrechterhalten werden.

In einer Ausgestaltung der Aktualisierungseinheit ist diese derart ausgestaltet ist, dass beim Aktualisieren der Vergleichswerte das Bilden eines Mittelwertes aus einer Anzahl von Wertesätzen erfolgt, die zuvor zu einer Zuordnung der physiologischen Signale, die sie repräsentieren, zu der zu aktualisierenden Ereignisklasse geführt haben. Die Vorrichtung umfasst somit vorzugsweise eine Mittelungseinheit für Wertesätze. Das Aktualisieren erfolgt dann anhand des so gebildeten Mittelwertes. Durch das Bilden des Mittelwertes kann verhindert werden, dass ein Aktualisieren einer Ereignisklasse allein anhand eines Signals erfolgt, dass zwar noch zur entsprechenden Ereignisklasse gehört, jedoch im Vergleich zu anderen dieser Klasse zugeordneten Signalen am Rande des Bereiches der zugehörigen Signalformen liegt. Das Klassifizieren anhand eines derartigen Signals könnte dazu führen, dass die aktualisierten Vergleichswerte die tatsächliche Änderung in der für die Ereignisklasse typischen Signalform nicht adäquat widerspiegeln.

In einer alternativen Ausgestaltung der Aktualisierungseinheit ist diese derart ausgestaltet, dass beim Aktualisieren der Vergleichswerte ein exponentielles Gewichten einer Anzahl von Wertesätzen, die zuvor zu einer Zuordnung der entsprechenden physiologischen Signale zu der zu aktualisierenden Ereignisklasse geführt haben, erfolgt. Die Vorrichtung umfasst somit eine Wertungseinheit für jeweils ein erfasstes physiologisches Signal charakterisierende Wertsätze. Das Aktualisieren erfolgt dann anhand der exponentiell gewichteten Wertesätze. In dieser Ausgestaltung können alle in der Vergangenheit in die Ereignisklasse zugeordneten Wertesätze Berücksichtigung finden.

Unabhängig von der Ausgestaltung der Aktualisierungseinheit kann das Aktualisieren einer Ereignisklasse nach dem Zuordnen des n-ten Wertesatzes zu dieser Ereignisklasse erfolgen. Dabei kann n auch den Wert Eins haben, d.h. das Aktualisieren erfolgt kontinuierlich mit jedem Wertesatz, welcher der entsprechenden Ereignisklasse zugeordnet wird. Die Wahl des Wertes für n, d.h. die Häufigkeit der Aktualisierung, kann dabei im Hinblick auf das Langzeitverhalten der Ereignisklassen erfolgen. Auch können für verschiedene Ereignisklassen verschiedenen Werte für n vorhanden sein, so dass die Aktualisierungshäufigkeit besonders gut an unterschiedliche Erfordernisse verschiedener Ereignisklassen angepasst werden kann.

In einer Weiterbildung umfasst die erfindungsgemäße Vorrichtung außerdem:
- einen Signaleingang zum Eingeben eines physiologischen Signals; eine mit dem Signaleingang zum Empfang des physiologischen Signals verbundene Transformationseinheit, die derart zum Durchführen einer Transformation des physiologischen Signals ausgestaltet ist, dass sie als Ausgangssignal eine Anzahl das physiologische Signal repräsentierender und auf der Transformation beruhender Werte ausgibt;
wobei das probabilistische neuronale Netzwerk mit der Transformationseinheit zum Empfang der Werte als Wertesatz verbunden ist.

In einer bevorzugten Ausgestaltung dieser Weiterbildung ist die Transformationseinheit zum Durchführen der Transformation anhand von Wavelets und einer die auszugebenden Werte unter Verwendung der Wavelets bestimmenden Transformationsregel ausgestaltet. Die Wavelet-Transformation ist einfach durchzuführen und ermöglicht es, Signale mit relativ wenigen Werten (in Form von Koeffizienten) zu repräsentieren. Gleichzeitig beleibt bei der Wavelet-Transformation genügend Information über das Signal erhalten, um eine zuverlässige Klassifikation im probabilistischen neuronalen Netzwerk zu gewährleisten. Zudem bietet die Wavelet-Transformation die Möglichkeit, die Transformation innerhalb der für das Berechnen der Transformation bestehenden Grenzen an das effektive Erkennen einzelner Ereignisklassen anzupassen. Vorzugsweise enthalten die durch die Wavelet-Transformation gewonnenen Werte neben Werten, die ein Stammwavelet beschreiben, zusätzlich Skalierungswerte und Translationswerte, die bezogen auf ein jeweiliges Stammwavelet die Form des Eingangssignals (physiologisches Signal) charakterisieren.

Weitere Merkmale, Eigenschaften und Vorteile der vorliegenden Erfindung ergeben sich aus der nachfolgenden Beschreibung eines Ausführungsbeispiels unter Bezugnahme auf die beiliegenden Zeichnungen.

Figur 1 zeigt ein Ausführungsbeispiel für die vorliegende Erfindung.

Figur 2 zeigt die Aktualisierungseinheit des Ausführungsbeispiels.

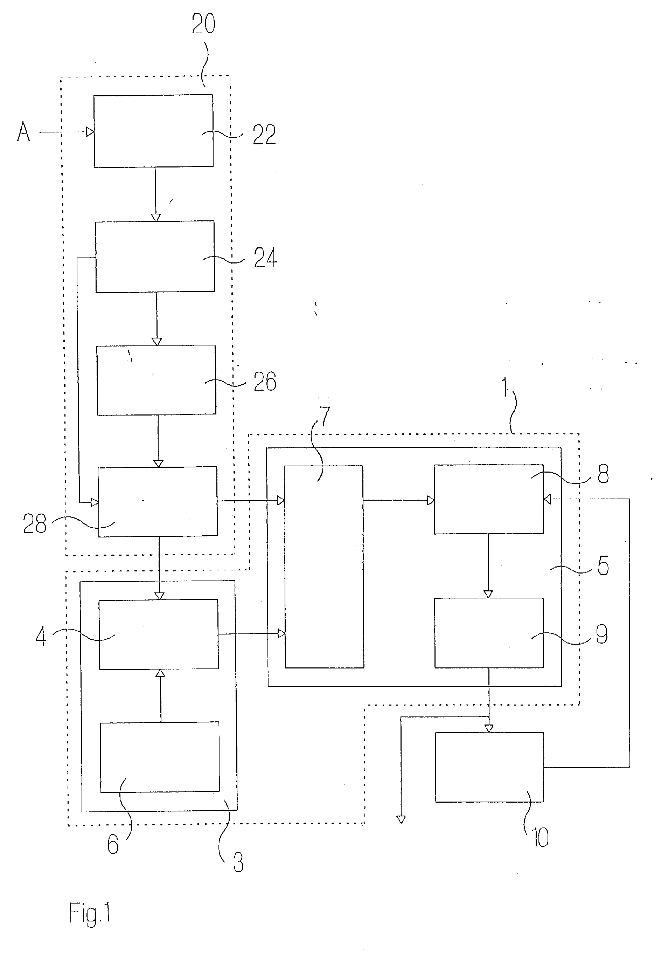

Im in Fig.1 dargestellten Ausführungsbeispiel wiest die Vorrichtung zur Klassifikation physiologischer Ereignisse eine Klassifikationseinheit 1 auf, die eine Transformationseinheit 3 sowie ein probabilistisches neuronales Netzwerk 5 umfasst, das mit der Transformationseinheit 3 zum Empfang von Koeffizienten (d.h. Werten), welche ein in die Transformationseinheit 3 eingehendes und ggf. aufbereitetes physiologisches Signal repräsentieren, verbunden ist. Außerdem ist der Transformationseinheit 3 im vorliegenden Ausführungsbeispiel eine Signalaufbereitungseinheit 20 vorgeschaltet, die einen Anti-Alias-Filter 22, einen Breitband-Analog-Digital-Wandler 24, im Folgenden kurz A/D-Wandler genannt, eine Detektionsstufe 26 zum Detektieren eines physiologischen Ereignisses, sowie eine kombinierte Justier/Normierstufe 28 umfasst, die von einem eingehenden physiologischen Eingangssignal A in dieser Reigenfolge durchlaufen werden, und die zum Ausgeben eines aufbereiteten physiologischen Signals mit der Transformationseinheit verbunden ist. Außerdem umfasst die Vorrichtung zum Klassifizieren physiologischer Ereignisse eine Aktualisierungseinheit 10, die mit dem probabilistischen neuronalen Netzwerk 5 zum Empfang dessen Ausgangssignals und zum Aktualisieren des probabilistischen neuronalen Netzwerks 5 auf der Basis des Ausgangssignals verbunden ist.

Die in der Einleitung erwähnte Mittelungseinheit oder die Wertungseinheit sind vorzugsweise Bestandteile der Aktualisierungseinheit und in Figur 1 nicht weiter dargestellt.

Im Folgenden wird die in der Signalaufbereitungseinheit 20 vorgenommene Signalaufbereitung des physiologischen Eingangssignals A, das im vorliegenden Ausführungsbeispiel ein intrakardiales Elektrogramm (IEGM) ist, wie es bspw. mittels eines Herzschrittmachers aufzunehmen ist, kurz erläutert. Jedoch sei darauf hingewiesen, dass die mit der vorliegenden Erfindung klassifizierbaren physiologischen Signale nicht auf intrakardiale Elektrogramme beschränkt sind.

Im Anti-Alias-Filter 22 erfolgt ein Filtern des IEGMs mittels eines Anti-Alias-Tiefpassfilters sowie ein geeignetes Verstärken und/oder Skalieren des IEGMs. Wie für Systeme abgetasteter Daten (sampled data systems) bekannt ist, unterdrückt der Filter Signalkomponenten, die bei Frequenzen oberhalb der halben Abtastrate auftreten können und von den nachfolgenden Signalverarbeitungsschritten aufgeprägt werden. Um die Genauigkeit und die Morphologie der Signalform des IEGMs zu erhalten, erfolgt darüber hinaus kein weiteres Filtern.

Das gefilterte IEGM wird von Anti-Alias-Filter 22 an den A/D-Wandler 24 weitergegeben, der ein konventioneller Analog-Digital-Wandler mit einer schrittweise linearen Beziehung zwischen dem Eingangssignal und dem Ausgangssignal ist. Die Abtastrate und die Auflösung des Ausgangssignals sind an die Anforderungen des Klassifizierungsprozesses angepasst. Im Allgemeinen liegen sie bei 1024 Hz oder darunter bzw. bei 8 Bit oder darüber. Abhängig von den Anforderungen können verschiedene A/D-Wandler-Architekturen zur Anwendung kommen, einschließlich des sog. "Ein-Bit-Designs" (One-Bit-Design). In speziellen Fällen, in denen Eingangssignale mit einem großen Dynamikbereich vorliegen, kann das Verwenden nichtlinearer (kompandierender, d.h. das Signal komprimierender und anschließend wieder expandierender) A/D-Wandlerstrukturen vorteilhaft sein. Das gewandelte IEGM wird vom A/D-Wandler 24 als Ausgangssignal an die Detektionsstufe 26 sowie an die Justier/Normierstufe 28 weitergeleitet.

In der Detektionsstufe 26 erfolgt die Detektion eines Ereignisses auf der Basis einer von Ereignis zu Ereignis ratenadaptiven Schwellenwertbetrachtung. Das Ergebnis der Detektion zeigt eine Aktivität der Signalform des Eingangssignals an. Wenn die Detektionsstufe 26 ein Ereignis detektiert, gibt sie ein Triggersignal (Auslösesignal) an die Justier/Normierstufe 28 aus, welches ein Justieren und/oder Normieren des physiologischen Signals auslöst.

Wenn die Justier/Normierstufe 28 ein Triggersignal von der Detektionsstufe 26 empfängt, so wird das zugrunde liegende IEGM in einem Ereignisfenster mit einer vorgegebenen Fensterweite, die im Allgemeinen 64 Abtastschritte beträgt, erfasst und im Fenster zentriert. Das Fenster ist an den erwarteten Ereignistyp angepasst. Es erfolgt außerdem ein Ermitteln des Zeitintervalls vom zuletzt detektierten Ereignis bis zum aktuellen Ereignis sowie eine Normierung der Signalform auf eine standardisierte Spitze-zu-Spitze-Amplitude anhand eines Normierungsfaktors, um ein normiertes Ereignissignal zu erhalten. Das Zeitintervall und den Normierungsfaktor gibt die Justier/Normierstufe 28 an das probabilistische neuronale Netzwerk 5 weiter, wohingegen sie das im Fenster zentrierte und nprmierte Ereignissignal an Transformationseinheit 3 weitergibt.

Die Transformationseinheit 3 nimmt eine Wavelet-Transformation des zentrierten und normierten Ereignissignals vor, die eine Anzahl das Signal repräsentierender Koeffizienten zum Ergebnis hat. Die Wavelet-Transformation ist ein wohlbekanntes Verfahren zum kompakten Darstellen beliebiger Signale. Dabei erfolgt die Transformation eines Signals mit Hilfe von Referenz-Wavelets und einem Berechnungsverfahren, das angibt, wie die Referenz-Wavelets mit dem Signal zu verrechnen sind. Die Details der Transformation können innerhalb der durch das Berechnungsumfeld gegebenen mathematischen Grenzen derart gewählt werden, dass sie sich für bestimmte Signalklassen sehr effektiv einsetzen lässt. Im vorliegenden Ausführungsbeispiel, das für den Einsatz in einer implantierbaren medizinischen Vorrichtung gedacht ist, ermöglicht die Wavelet-Transformation, ein Ereignisfenster mit einer Fensterweite von 64 Abtastschritten (64-Koeffizienten DWT) mit weniger als 16 Wavelet-Transformationskoeffizienten darzustellen und gleichzeitig genügend Information des Signals zu erhalten, um eine verlässliche Ereignisklassifikation im probabilistischen neuronalen Netzwerk 5 zu gewährleisten.

Zum Durchführen der Wavelet-Transformation umfasst die Transformationseinheit 3 einen Waveletspeicher 6, in dem die Referenz-Wavelets gespeichert sind, und eine Recheneinheit 4, die mit der Justier/Normierstufe 28 zum Empfang des im Fenster zentrierten und normierten Ereignissignals und mit dem Waveletspeicher 6 zum Empfang der Referenz-Wavelets verbunden ist. In der Recheneinheit 4 findet das Berechnen der Koeffizienten, d.h. die eigentliche Wavelet-Transformation statt.

Es existiert eine Anzahl Berechnungsverfahren, die zum Berechnen der Wavelet-Transformation geeignet sind. Ebenso existiert eine Vielzahl geeigneter Referenz-Wavelets. Für das Berechnen der Wavelet-Transformation in der Recheneinheit 4 kann der verwendete Satz Referenz-Wavelets bspw. im Hinblick auf die erzielbare Rechenleistung ausgewählt werden. Beim Auswählen des Berechnungsverfahrens und der Referenz-Wavelets ist jedoch vorzugsweise darauf zu achten, dass beim Berechnen der Wavelet-Transformation in der Recheneinheit 4 dasselbe Berechnungsverfahren und derselbe Satz Referenz-Wavelets zur Anwendung kommen, wie sie beim Berechnen der Vergleichskoeffizienten (siehe weiter unten) zur Anwendung gekommen sind.

Die Recheneinheit 4 gibt das Ergebnis der Wavelet-Transformation, also die Wavelet-Transformationskoeffizienten, als Satz von Koeffizienten an das probabilistische neuronale Netzwerk 5 (im Folgenden PNN abgekürzt) aus.

Das PNN 5 umfasst eine PNN-Struktur 8, eine Eingabeschicht 7 (input layer) sowie eine Ausgabeschicht oder Summationseinheit 9 (output layer). Die PNN-Struktur 8 weist eine Anzahl innerer Knoten auf und ist mit der Eingabeschicht 7 (input layer), die eine Anzahl Eingabeknoten aufweist, sowie mit der Summationseinheit 9, die eine Anzahl Ausgabeknoten aufweist, verbunden.

Die inneren Knoten der PNN-Struktur 8 enthalten jeweils einen bestimmten Koeffizientenvektor, der Vergleichskoeffizienten als ein Satz von Vergleichswerten, ein bestimmtes Vergleichszeitintervall sowie einen bestimmten Vergleichsnormierungsfaktor enthält und eine bestimmte Klasse von Ereignissen charakterisiert. Im vorliegenden Ausführungsbeispiel enthält die PNN-Struktur 8 für jede Klasse genau einen Knoten, es ist jedoch auch möglich einer Klasse mehrere innere Knoten mit jeweils leicht unterschiedlichen Koeffizientenvektoren zuzuordnen, so dass eine Klasse von einem Knoten-Cluster repräsentiert wird.

Die Koeffizientenvektoren werden üblicherweise vorab aus einer Mehrzahl von Signalen mit der für die Ereignisklasse typischen Signalform extrahiert.

Aufgabe der Eingabeschicht 7 des PNN 5 ist es, von der Transformationseinheit 3 die Koeffizienten und von der Justier/Normierstufe 28 das Zeitintervall und den Normierungsfaktor des aktuellen IEGMs zu empfangen und gleichmäßig über die inneren Knoten der PNN-Struktur 8 zu verteilen.

In den inneren Knoten werden die jeweiligen Koeffizientenvektoren mit einem Signalvektor, der aus den von der Transformationseinheit 3 empfangenen Koeffizienten, sowie dem Zeitintervall und dem Normierungsfaktor des aktuellen IEGMs gebildet ist, verglichen, indem die Differenz aus Signalvektor und Koeffizientenvektor gebildet wird. Außerdem werden den ermittelten Vektordiffererizen Wahrscheinlichkeitswerte zugeordnet, wobei der Wahrscheinlichkeitswert umso größer ist, je geringer die Vektordifferenz ausfällt. Das Bestimmen der Wahrscheinlichkeitswerte kann eine gaußförmige Transferfunktion mit wählbarer Standardabweichung Sigma (die den Abstand der Wendepunkte der Kurve vom Kurvenzentrum angibt) einschließen. Die wählbare Standardabweichung ermöglicht es, die Grenzen, d.h. die maximal zulässige Abweichung von der jeweiligen Standardsignalform einer Klasse festzulegen.

Die PNN-Struktur 8 ist zum weitergeben des Signalvektors sowie der für den Signalvektor ermittelten Wahrscheinlichkeitswerte mit der Summationseinheit 9 verbunden.

Die Summationseinheit 9 besitzt genau einen Ausgangsknoten für jede Ereignisklasse, für deren Erkennen die erfindungsgemäße Vorrichtung ausgestaltet ist. Der Ausgangsknoten empfängt den für die jeweilige Ereignisklasse ermittelten Wahrscheinlichkeitswert des Signalvektors. Falls einer Ereignisklasse mehrere innere Knoten zugeordnet sind, empfängt der entsprechende Ausgangsknoten alle Wahrscheinlichkeitswerte dieser inneren Knoten und berechnet den Mittelwert der entsprechenden Wahrscheinlichkeitswerte. In beiden Fällen stellt der Wahrscheinlichkeitswert eines Ausgangsknotens der Summationseinheit 9 die Wahrscheinlichkeit dafür dar, dass das das IEGM bzw. das auslösende Ereignis der durch den Ausgangsknoten repräsentierten Klasse angehört. Derjenigen Klasse, die in der Summationseinheit 9 den höchsten Wahrscheinlichkeitswert aufweist, wird das das IEGM auslösende Ereignis zugeordnet, sofern dieser Wahrscheinlichkeitswert eine Klassifizierungsschwelle überschreitet. Überschreitet er die Klassifizierungsschwelle nicht, so wird das Ereignis als unbekannt klassifiziert und ggf. dazu verwendet, eine Anpassung der PNN-Struktur auszulösen, die zum Erkennen einer neuen Ereignisklasse führt. Die Summationseinheit 9 gibt schließlich den Signalvektor und die Ereignisklasse, der er zugeordnet worden ist, als Ergebnis der Klassifikation aus. Der Signalvektor und die Ereignisklasse, der er zugeordnet worden ist, wird von der Summationseinheit 9 außerdem an die Aktualisierungseinheit 10 weitergegeben.

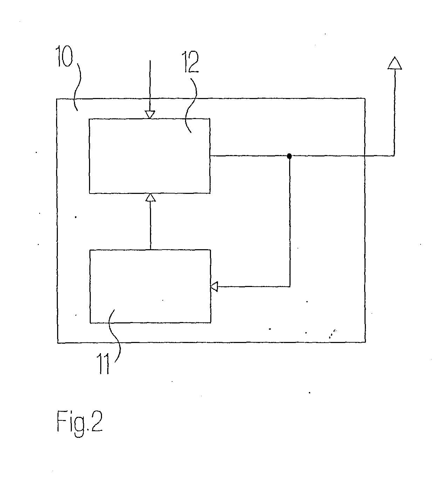

Die Aktualisierungseinheit 10 (siehe Fig. 2) umfasst einen Speicher 11, in dem der gerade aktuelle (d.h. bisher verwendete) Koeffizientenvektor für jeden inneren Knoten der PNN-Struktur 8 gespeichert ist. Daneben umfasst sie eine Kombinationseinheit 12, die mit dem PNN 5 zum Empfang des Signalvektors und der Ereignisklasse, der er zugeordnet worden ist, sowie mit dem Speicher 11 zum Empfang des entsprechenden aktuellen Koeffizientenvektors verbunden ist.

In der Kombinationseinheit 12 erfolgt das Bestimmen des neuen, d.h. aktualisierten, Koeffizientenvektors des inneren Knotens anhand des empfangenen Signalvektors und des im Speicher 11 gespeicherten aktuellen Koeffizientenvektors. Dazu erfolgt eine Multiplikation der im Signalvektor enthaltenen Koeffizienten mit einem Faktor α und eine Multiplikation der im aktuellen Koeffizientenvektor enthaltenen Koeffizienten mit einem Faktor (1 - α). Anschließend erfolgt das Ermitteln des neuen (d.h. aktualisierten) Koeffizientenvektors anhand der beiden so multiplizierten Vektoren. Dabei stellt jeder neue (aktualisierte) Koeffizient die Summe aus dem entsprechenden, mit dem mit dem Faktor (1 - α) multiplizierten Koeffizienten des aktuellen Koeffizientenvektors und dem entsprechenden, mit dem Faktor α multiplizierten Koeffizienten des Signalvektors dar. Enthalten Signalvektor und Koeffizientenvektor bspw. jeweils 16 Koeffizienten, so ist der erste neue Koeffizient des neuen Koeffizientenvektors durch die Summe des mit dem Faktor α multiplizierten ersten Koeffizienten des Signalvektors und des mit dem Faktor (1 - α) multiplizierten ersten Koeffizienten des aktuellen Koeffizientenvektors gegeben, der zweite neue Koeffizient des neuen Koeffizientenvektors durch die Summe des mit dem Faktor α multiplizierten zweiten Koeffizienten des Signalvektors und des mit dem Faktor (1 - α) multiplizierten zweiten Koeffizienten des aktuellen Koeffizientenvektors u.s.w.

Die beschriebene Vorgehensweise führt dazu, dass in jedem aktualisierten Koeffizientenvektor die Koeffizienten der zuvor dem entsprechenden inneren Knoten zugeordneten Signalvektoren mit einer exponentiellen Gewichtung berücksichtigt sind. Um die physiologischen Gegebenheiten adäquat zu berücksichtigen, sollte der Faktor α deutlich keiner als 1 sein. Der aktualisierte Koeffizientenvektor wird schließlich an die PNN-Struktur 8 und an den Speicher 11 weitergegeben, wo er den bisherigen Koeffizientenvektor des entsprechenden Knotens ersetzt.

Alternativ zum exponentiellen Gewichten der Koeffizienten aus den zuvor für die Ereignisklasse ermittelten Signalvektoren kann auch das Bilden eines Mittelwertes erfolgen. Jeder neue Koeffizient des neuen Koeffizientenvektors stellt dann einen Mittelwert aus den entsprechenden Koeffizienten einer Anzahl zuvor dem inneren Knoten zugeordneter Signalvektoren dar. Die Anzahl kann entweder fest vorgegeben sein oder alle Signalvektoren umfassen, die innerhalb eines vorgegeben, physiologisch sinnvollen Zeitabschnittes der entsprechenden Ereignisklasse, d.h. dem entsprechenden innern Knoten zugeordnet worden sind. Zum Speichern der Signalvektoren umfasst die Aktualisierungseinheit 10 dann einen weiteren Speicher, der bspw. auch als Speicherabschnitt des Speichers 11 ausgebildet sein kann.

Das beschriebene Berechnen des aktualisierten Koeffizientenvektors eines inneren Knotens sowie das Ersetzen des bisherigen Koeffizientenvektors dieses inneren Knotens geschehen im vorliegenden Ausführungsbeispiel mit jedem Signalvektor, der dem Knoten zugeordnet wird. Alternativ kann das Berechnen und/oder Ersetzen jedoch auch nur nach jedem n-ten, bspw. nach jedem 10-ten, Signalvektor, erfolgen, welcher dem inneren Knoten zugeordnet wird.

Obwohl die Aktualisierungseinheit im vorliegenden Ausführungsbeispiel außerhalb der Klassifikationseinheit 1 angeordnet ist, kann sie auch in die Klassifikationseinheit 1, bspw. in die Summationseinheit 9, integriert sein.

Die vorliegende Erfindung kann sowohl in Form von Hardware als auch in Form von Software implementiert werden.

## Patentansprüche

1. Vorrichtung zur Klassifikation physiologischer Ereignisse anhand von die physiologischen Ereignisse darstellenden oder repräsentierenden physiologischen Signalen mit Hilfe eines probabilistischen neuronalen Netzwerks (5), umfassend:
- ein zum Empfang eines das physiologische Signal repräsentierenden Wertesatzes ausgestaltetes probabilistisches neuronales Netzwerk (5), das eine Anzahl physiologische Ereignisse repräsentierender Ereignisklassen definiert, die jeweils durch eine Anzahl von Vergleichswerten bestimmt sind, und das dazu ausgestaltet ist, anhand des Vergleichs des Wertesatzes mit den Vergleichswerten eine Zuordnung des von dem Wertesatz repräsentierten physiologischen Signals zu einer der Ereignisklassen vorzunehmen, und
- eine mit dem probabilistischen neuronalen Netzwerk (5) verbundene Aktualisierungseinheit (10) zum Aktualisieren der Vergleichswerte einer. Ereignisklasse anhand des Wertesatzes mindestens eines physiologischen Signals, das bei einer vorangegangen Zuordnung dieser Ereignisklasse zugeordnet worden ist.

2. Vorrichtung nach Anspruch 1, bei der die Aktualisierungseinheit (10) derart ausgestaltet ist, dass beim Aktualisieren der Vergleichswerte das Bilden eines Mittelwertes aus einer Anzahl von Wertesätzen erfolgt, die zuvor zu einer Zuordnung der physiologischen Signale, die sie repräsentieren, zu der zu aktualisierenden Ereignisklasse geführt haben, und bei der das Aktualisieren anhand des so gebildeten Mittelwertes erfolgt.

3. Vorrichtung nach Anspruch 1, bei der die Aktualisierungseinheit (10) derart ausgestaltet ist, dass beim Aktualisieren der Vergleichswerte ein exponentielles Gewichten einer Anzahl von Wertesätzen, die zuvor zu einer Zuordnung der physiologischen Signale, die sie repräsentieren, zu der zu aktualisierenden Ereignisklasse geführt haben, erfolgt, und bei der das Aktualisieren anhand der exponentiell gewichteten Wertesätze erfolgt.

4. Vorrichtung nach einem der Ansprüche 1 bis 3, bei der die Aktualisierungseinheit (10) derart ausgestaltet ist, dass das Aktualisieren einer Ereignisklasse nach dem Zuordnen eines n-ten Wertesatzes zu dieser Ereignisklasse erfolgt, wobei dies eine vorgegebene Anzahl von Wertsätzen definiert.

5. Vorrichtung nach Anspruch 4, **dadurch gekennzeichnet, dass** unterschiedlichen Ereignisklassen unterschiedliche Werte für n zuzuordnen sind.

6. Vorrichtung nach einem der vorangehenden Ansprüche, die außerdem umfasst:
- einen Signaleingang zum Eingeben eines physiologischen Signals;
- eine mit dem Signaleingang zum Empfang des physiologischen Signals verbundene Transformationseinheit (3), die derart zum Durchführen einer Transformation des physiologischen Signals ausgestaltet ist, dass sie als Ausgangssignal eine Anzahl von das physiologische Signal repräsentierenden und auf der Transformation beruhenden Werten ausgibt;
wobei das probabilistische neuronale Netzwerk (5) mit der Transformationseinheit (3) zum Empfang der Werte als Wertesatz verbunden ist.

7. Vorrichtung nach Anspruch 6, **dadurch gekennzeichnet, dass** die Transformationseinheit (3) zum Durchführen der Transformation anhand von Wavelets und einer die auszugebenden Werte unter Verwendung der Wavelets bestimmenden Transformationsregel ausgestaltet ist.

8. Implantierbares medizinisches Gerät, **dadurch gekennzeichnet, dass** es mit einer Vorrichtung zur Klassifikation physiologischer Ereignisse nach einem der Ansprüche 1 bis 7 ausgestattet ist.

9. Implantierbares medizinisches Gerät nach Anspruch 8, **gekennzeichnet durch** seine Ausgestaltung als Herzschrittmacher oder Defibrillator.
